# EUROPEAN PATENT SPECIFICATION

(11) **EP 3 944 830 B1**
(45) Date of publication and mention of the grant of the patent: **26.02.2025**
(21) Application number: 21165830.7
(22) Date of filing: 30.03.2021
(51) Int. Cl.: A61B 18/14

(54) **SYSTEM COMPRISING A FOCAL CATHETER HAVING CONTACT-FORCE AND TEMPERATURE SENSORS**
SYSTEM MIT EINEM FOKALEN KATHETER MIT KONTAKTKRAFT UND TEMPERATURSENSOREN
SYSTÈME COMPRENANT UN CATHÉTER FOCAL DOTÉ DE CAPTEURS DE FORCE DE CONTACT ET DE TEMPÉRATURE

(30) Priority: 28.07.2020 US 202016940802
(43) Date of publication of application: 02.02.2022
(73) Proprietor: Biosense Webster (Israel) Ltd, Yokneam, 2066717 (IL)
(72) Inventor: ALTMANN, Andres Claudio, 2066717 Yokneam (IL); GOVARI, Assaf, 2066717 Yokneam (IL)
(74) Representative: Carpmaels & Ransford LLP

(56) References cited:
- EP-B1- 3 476 344
- WO-A1-2015/055112
- WO-A1-2015/103539
- US-A1- 2017 189 103

## Description

### FIELD OF THE INVENTION

The present invention relates generally to irreversible electroporation (IRE) procedures, and particularly to systems for improving control of IRE pulses applied to tissue.

### BACKGROUND OF THE INVENTION

Various systems have been proposed for radiofrequency (RF) ablation. For example, US Patent Application Publication No. 2017/189103 describes an RF catheter probe comprising an insertion tube, and a distal end with a distal electrode, a force sensor to detect force on the distal electrode, and an irrigated electrode mounted on a coupling member of the force sensor, which has a tubular form surrounding a central space occupied by components, including force sensing coils. A fluid diverter that passes fluid to the proximal irrigated electrode is configured as an insert or an integrated projection of the coupling member. The fluid diverter has a proximal entry opening and a distal exit opening connected by a fluid passage with at least a radial branch and at least an axial branch. The irrigated electrode is mounted over the distal exit opening to receive fluid from the fluid passage.

International Patent Application Publication WO2015/055112 describes a medical catheter and a radio frequency treatment system having said catheter. The catheter is provided with an annular end portion having a plurality of electrodes. The annular end portion is further provided with a force sensor. The force sensor is used to measure the contact force between the annular end portion and other objects, and assists in controlling contact between the annular end portion and vascular walls or organ surfaces.

European Patent Application Publication EP3476344A1 describes a catheter system comprising an at least partially flexible catheter body, at least a ring electrode, and at least a strain gauge. The ring electrode surrounds at least a portion of the flexible catheter body. The strain gauge is allocated to the ring electrode and the strain gauge is configured to measure a deformation of the flexible catheter body at a position allocated to the ring electrode to detect a contact between the ring electrode and tissue.

Turning to irreversible electroporation, various techniques for controlling irreversible electroporation (IRE) procedures are known in the art.

For example, European Patent Application 3459480 describes an apparatus for localizing an electrical field for electroporation of tissue. The apparatus includes a pulsed DC electrical power supply and at least one catheter tip and electrode assembly configured for endocardial placement, such that electrodes positioned at separate endocardial locations allow development of an electrical field between the electrodes to effect electroporation of tissue in the electrical field.

U.S. Patent Application Publication No. 2018/0214202 described methods, systems, and devices for enhancing the efficiency and efficacy of energy delivery and tissue mapping. One system includes a treatment element having a plurality of electrodes and an energy generator that is configured to deliver electric energy pulses to the electrodes in a variety of patterns.
WO 2015/103539 A1 describes endovascular catheters, configured for delivery to a carotid artery from a subclavian artery approach, for assessing, and treating patients having sympathetically mediated disease, involving augmented peripheral chemoreflex and heightened sympathetic tone by reducing chemosensor input to the nervous system via carotid body ablation. Pressure or force sensors may be incorporated into any of the catheters, for example they could be mounted to a flex circuit proximate an ablation element, and could be used to verify contact or indicate contact force. Diverging arms with open/close actuation could be actuated to a position that corresponds to a particular contact pressure range. Alternatively, a catheter could be "pushed" against the wall until contact pressure reaches a desired level. Alternatively, a baseline pressure may be chosen when a desirable contact force is visually confirmed, for example vessel distension caused by ablation element contact force may visually appear using an imaging modality such as angiography. A change of pressure or force, within an acceptable range from the baseline, measured by the sensors may indicate appropriate contact force and deviation from this range could indicate an inappropriate contact force. A computer algorithm that controls delivery of ablation energy may discontinue energy delivery if contact force deviates from the appropriate range.

### SUMMARY OF THE INVENTION

An embodiment of the present invention that is described herein provides a system comprising a focal catheter including an insertion tube, first and second electrodes, and a contact-force sensor. The insertion tube is configured to insert the catheter into a patient body. The first and second electrodes are coupled to a distal-end of the catheter at a predefined distance from one another, and are configured to: (i) receive, via the insertion tube, one or more irreversible electroporation (IRE) pulses, and (ii) apply the IRE pulses, between the first and second electrodes, to tissue of the patient body. The contact-force sensor is disposed between the first and second electrodes and is configured to produce an electrical signal indicative of a contact force applied between the distal-end and the tissue. The system includes an IRE pulse generator (IPG) configured to supply the one or more IRE pulses; and a processor, which is configured, based on the electrical signal received from the contact-force sensor, to control the IPG to supply of the one or more IRE pulses to the first and second electrodes.

In some embodiments, the catheter includes at least a temperature sensor, which is coupled to the distal-end, and is configured to produce a temperature signal, indicative of a measured temperature of at least one of the distal-end and the tissue. In yet other embodiments, the temperature sensor includes a thermocouple.

In an embodiment, the processor is configured to: (i) hold a temperature threshold, and (ii) control the supply of the one or more IRE pulses to the first and second electrodes, based on a comparison between the measured temperature and the temperature threshold. In another embodiment, the catheter includes a focal catheter. In yet another embodiment, the first and second electrodes are coupled along an axis of the catheter.

There is additionally provided, in accordance with an embodiment of the present invention, a method for producing a focal catheter according to appended claim 6.

### BRIEF DESCRIPTION OF THE DRAWINGS

The present invention will be more fully understood from the following detailed description of the embodiments thereof, taken together with the drawings in which:
Fig. 1 is a schematic, pictorial illustration of a catheter-based position-tracking and irreversible electroporation (IRE) ablation system, in accordance with an exemplary embodiment of the present invention;
Fig. 2 is a flow chart that schematically illustrates a method for producing an IRE focal catheter having contact-force and temperature sensors, in accordance with an exemplary embodiment of the present invention; and
Fig. 3 is a flow chart that schematically illustrates a method for controlling one or more IRE pulses applied to tissue, based on signals indicative of contact-force and temperature measured during IRE ablation procedure, in accordance with an exemplary embodiment of the present invention.

### DETAILED DESCRIPTION OF EMBODIMENTS

### OVERVIEW

Irreversible electroporation (IRE) may be used, for example, for treating arrhythmia by ablating tissue cells using high-voltage applied pulses. Cellular destruction occurs when the transmembrane potential exceeds a threshold, leading to cell death and formation of a lesion. In IRE-based ablation procedures, high-voltage bipolar electrical pulses are applied, for example, to a pair of electrodes in contact with tissue to be ablated, so as to form a lesion between the electrodes, and thereby to treat arrhythmia in a patient heart.

Embodiments of the present invention that are described hereinbelow provide improved techniques for controlling IRE ablation by controlling one or more IRE pulses applied to tissue, also referred to herein as target tissue, at an IRE ablation site.

In some cases, a focal catheter may be required to carry out an ablation procedure. In principle, in unipolar radiofrequency (RF) ablation, the focal catheter may comprise (i) a contact-force sensing device for estimating the contact force applied between the catheter and the target tissue, (ii) a large distal electrode for ablating the target tissue, and (iii) one or more ring electrodes for diagnostic measurements. This configuration, however, cannot be used for applying high-voltage bipolar IRE pulses, for example because: (a) the high proximity between the electrodes prevents applying such high-voltage bipolar pulses, and (b) at least one of the electrodes may be overheated in response to applying the high-voltage bipolar pulses.

According to the invention, a system configured to carry out a controlled IRE ablation comprises a focal catheter having an insertion tube, which is configured to insert the catheter into an ablation site in the patient's body, such as in the patient's heart. The catheter may comprise a pair of similar electrodes, also referred to herein as first and second electrodes, which are substantially wider than the aforementioned ring electrodes.

The first and second electrodes are coupled to the distal-end of the catheter at a predefined distance from one another, and are configured to: (i) receive, e.g., via the insertion tube, one or more IRE pulses produced by an IRE pulse generator (IPG), and (ii) apply the IRE pulses, between the first and second electrodes, to tissue at the ablation site.

The catheter comprises a contact-force sensor, which is disposed between the first and second electrodes and is configured to produce an electrical signal indicative of the contact force applied between the distal-end and the tissue.

In some embodiments, the catheter comprises one or more temperature sensors, which is coupled to the distal-end at one or more respective positions. Each temperature sensor is configured to produce a temperature signal, indicative of the measured temperature of at least one of the distal-end and the tissue.

In some embodiments, in an IRE ablation procedure, a physician inserts the distal-end into the ablation site and brings the first and second electrodes into contact with the target tissue. In some embodiments, during the IRE ablation, a processor of the system is configured to receive the signals indicative of the contact force applied between the distal-end and the target tissue, and the measured temperature. Based on the received signals, the processor is configured to assist the physician in controlling the IRE ablation, by controlling the contact force applied between the distal-end and the tissue, and parameters of the one or more pulses applied to the tissue. For example, (i) before applying one or more IRE pulses, the processor may alert the physician in case the contact between the distal-end and the tissue is not within a specified range of contact-force of the IRE procedure, (ii) during and after applying one or more pulses of IRE, the processor may check whether or not the temperature measured by the temperature sensors is within a specified range of temperatures of the IRE procedure.

In some embodiments, the processor may hold a temperature threshold and compare between the measured temperature and the temperature threshold, such that in case the measured temperature exceeds the temperature threshold, the processor may alert the physician to, or may autonomously, control the IPG to stop applying IRE pulses to the tissue.

The disclosed techniques improve the control of IRE ablation, and thereby, improve the patient safety and reduce the duration of IRE ablation procedures.

### SYSTEM DESCRIPTION

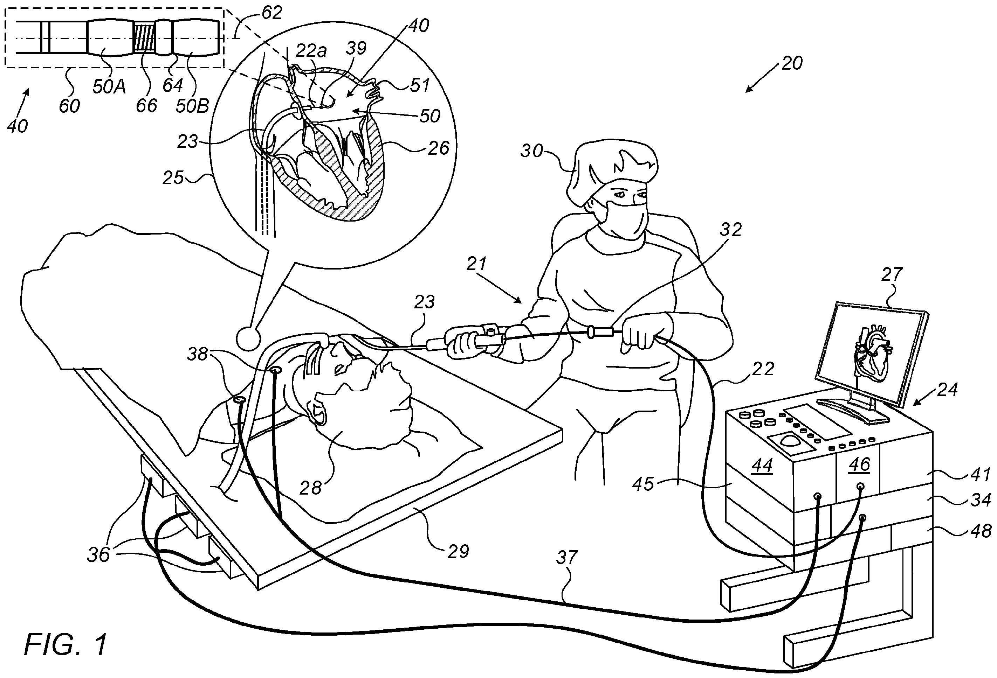

Fig. 1 is a schematic, pictorial illustration of a catheter 21 based position-tracking and irreversible electroporation (IRE) ablation system 20, in accordance with an embodiment of the present invention. Catheter 21 comprises a focal-type IRE ablation catheter.

In some embodiments, system 20 comprises a tip section 40, which is deflectable or non-deflectable, and is fitted at a distal-end 22a of a shaft 22 of catheter 21 with tip section 40 comprising multiple electrodes 50 as illustrated in inset 25.

In the embodiment described herein, electrodes 50 are configured for IRE ablation of tissue of left atrium of a heart 26, such as IRE ablation of an ostium 51 of a pulmonary vein in heart 26. Electrodes 50 may additionally be used to sense intra-cardiac (IC) electrocardiogram (ECG) signals. Note that the techniques disclosed herein are applicable, *mutatis mutandis,* to other sections (e.g., atrium or ventricle) of heart 26, and to other organs of a patient 28.

In some embodiments, the proximal end of catheter 21 is connected to a control console 24 (also referred to herein as a console 24) comprising an ablative power source, in the present example an IRE pulse generator (IPG) 45, which is configured to deliver peak power in the range of tens of kW. Console 24 comprises a switching box 46, which is configured to switch the power applied by IPG 45 to one or more selected pairs of electrodes 50. A sequenced IRE ablation protocol may be stored in a memory 48 of console 24.

In some embodiments, a physician 30 inserts distal-end 22a of shaft 22, e.g., using an insertion tube of shaft 22 that is configured for inserting catheter 21, through a sheath 23 into heart 26 of patient 28 lying on a table 29. Physician 30 navigates distal-end 22a of shaft 22 to a target location in heart 26 by manipulating shaft 22 using a manipulator 32 near the proximal end of catheter 21 and/or deflection from sheath 23. During the insertion of distal-end 22a, tip section 40 is maintained in a straightened configuration by sheath 23. By containing tip section 40 in a straightened configuration, sheath 23 also serves to minimize vascular trauma when physician 30 moves catheter 21, through the vasculature of patient 28, to the target location, such as an ablation site in heart 26.

Once distal-end 22a of shaft 22 has reached the ablation site, physician 30 retracts sheath 23 and, in case of a deflectable tip section, deflects tip section 40, and further manipulates shaft 22 to place electrodes 50 disposed over tip section 40 in contact with ostium 51 at the ablation site.

In some embodiments, electrodes 50 are connected by wires running, through the aforementioned insertion tube of shaft 22, to a processor 41, which is configured to control switching box 46 of interface circuits 44 in console 24.

Reference is now made to the inset 25. In some embodiments, distal-end 22a comprises a position sensor 39 of a position tracking system, which is coupled to distal-end 22a, e.g., at tip section 40. In the present example, position sensor 39 comprises a magnetic position sensor, but in other embodiments, any other suitable type of position sensor (e.g., other than magnetic-based) may be used. During navigation of distal-end 22a in heart 26, processor 41 of console 24 receives signals from magnetic position sensor 39 in response to magnetic fields from external field generators 36, for example, for the purpose of measuring the position of tip section 40 in heart 26 and, optionally, displaying the tracked position overlaid on the image of heart 26, on a display 27 of console 24.

Reference is now made back to the general view of Fig. 1. In some embodiments, magnetic field generators 36 are placed at known positions external to patient 28, e.g., below table 29. Console 24 also comprises a driver circuit 34, configured to drive magnetic field generators 36.

The method of position sensing using external magnetic fields is implemented in various medical applications, for example, in the CARTO^{™} system, produced by Biosense Webster Inc. (Irvine, Calif.) and is described in detail in U.S. Patent Nos. 5,391,199, 6,690,963, 6,484,118, 6,239,724, 6,618,612 and 6,332,089, in PCT Patent Publication WO 96/05768, and in U.S. Patent Application Publication Nos. 2002/0065455 A1, 2003/0120150 A1 and 2004/0068178 A1.

Typically, processor 41 of console 24 comprises a general-purpose processor of a general-purpose computer, with suitable front end and interface circuits 44 for receiving signals from catheter 21, as well as for applying ablation energy via catheter 21 in a left atrium of heart 26 and for controlling the other components of system 20. Processor 41 typically comprises a software in memory 48 of system 20, which is programmed to carry out the functions described herein. The software may be downloaded to the computer in electronic form, over a network, for example, or it may, alternatively or additionally, be provided and/or stored on non-transitory tangible media, such as magnetic, optical, or electronic memory.

### MONITORING AND CONTROLLING IRREVERSIBLE ELECTROPORATION PULSES APPLIED TO TISSUE

Irreversible electroporation (IRE), also referred to as Pulsed Field Ablation (PFA), may be used as a minimally invasive therapeutic modality to for forming a lesion (e.g., killing tissue cells) at the ablation site by applying high-voltage pulses to the tissue. In the present example, IRE pulses may be used for killing myocardium tissue cells in order to treat cardiac arrhythmia in heart 26. Cellular destruction occurs when the transmembrane potential exceeds a threshold, leading to cell death and thus the development of a tissue lesion. Therefore, of particular interest is the use of high-voltage bipolar electrical pulses, e.g., using a pair of electrodes 50 in contact with tissue at the ablation site, to generate high electric fields (e.g., above a certain threshold) to kill tissue cells located between the electrodes.

In the context of this disclosure, "bipolar" voltage pulse means a voltage pulse applied between two electrodes 50 of catheter 21 (as opposed, for example, to unipolar pulses that are applied, e.g., during a radio-frequency ablation, by a catheter electrode relative to some common ground electrode not located on the catheter).

To implement IRE ablation over a relatively large tissue region of heart 26, such as a circumference of an ostium of a pulmonary vein (PV) or any other suitable organ, it is necessary to use multiple pairs of electrodes 50 of catheter 21, or any other suitable type of IRE catheter, having multi electrodes 50 in tip section 40. To make the generated electric field as spatially uniform as possible over a large tissue region it is best to have pairs of electrodes 50 selected with overlapping fields, or at least fields adjacent to each other. However, there is a Joule heating component that occurs with the IRE generated fields, and this heating may damage the electrodes when multiple pairs of electrodes 50 are continuously used for delivering a sequence of IRE pulses.

In an embodiment, system 20 comprises surface electrodes 38, shown in the example of Fig. 1, as attached by wires running through a cable 37 to the chest and shoulder of patient 28. In some embodiments, surface electrodes 38 are configured to sense body-surface (BS) ECG signals in response to beats of heart 26. Acquisition of BS ECG signals may be carried out using conductive pads attached to the body surface or any other suitable technique. As shown in Fig. 1, surface electrodes 38 are attached to the chest and shoulder of patient 28, however, additional surface electrodes 38 may be attached to other organs of patient 28, such as limbs.

In some embodiments, electrodes 50 are configured to sense intra-cardiac (IC) ECG signals, and (e.g., at the same time) surface electrodes 38 are sensing the BS ECG signals. In other embodiments, sensing the IC ECG signals may be sufficient for performing the IRE ablation, so that surface electrodes 38 may be applied for other use cases.

In some embodiments, physician 30 may couple at least a pair of electrodes 50 to tissue, also referred to herein as target tissue, at the ablation site in heart 26. The target tissue is intended to be ablated by applying one or more IRE pulses via electrodes 50. Note that the IRE pulses may be applied to the target tissue multiple times, e.g., during different stages of the IRE ablation procedure.

Reference is now made back to an inset 60. According to the invention, , tip section 40, which is fitted at distal-end 22a of catheter 21, comprises a pair of first and second electrodes 50, referred to herein as electrodes 50A and 50B, respectively. Electrodes 50A and 50B are coupled to tip section 40 of distal-end 22a, at a predefined distance from one another.

In some embodiments, electrodes 50A and 50B are similar to one another, and are relatively wide, e.g., larger than about 1 mm along an axis 62 of tip section 40 of catheter 21. In the present example, electrodes 50A and 50B are disposed along axis 62, which is a longitudinal axis of catheter 21. In other embodiments, electrodes 50A and 50B may be disposed, relative to one another, at any suitable positions in distal-end 22a and may have any suitable predefined distance between one another. For example, at least one of electrodes 50A and 50B may be disposed at the edge of tip section 40.

In the context of the present disclosure and in the claims, the terms "about" or "approximately" for any numerical values or ranges indicate a suitable dimensional tolerance that allows the part or collection of components, or a physical parameters such as speed and time, to function for its intended purpose as described herein. More specifically, "about" or "approximately" may refer to the range of values ±20% of the recited value, e.g. "about 90%" may refer to the range of values from 71% to 99%.

Tip section 40 of distal-end 22a comprises a contact-force sensor 66, which is disposed between the first and second electrodes, in the present example, electrodes 50A and 50B, respectively. Contact-force sensor 66 is configured to produce an electrical signal indicative of a contact force applied between tip section 40 of distal-end 22a, and the aforementioned tissue located at the ablation site of heart 26.

In other embodiments, one or more contact-force sensors 66 may be coupled to tip section 40 of distal-end 22a, in addition to contact-force sensor 66 shown in inset 60, at any other suitable respective locations.

In some embodiments, tip section 40 of distal-end 22a comprises at least a temperature sensor 64, which is coupled to distal-end 22a, and is configured to produce a temperature signal, indicative of a measured temperature of at least one of distal-end 22a and the tissue ablated at the target location. In some embodiments, tip section 40 may comprise multiple temperature sensors 64 disposed at respective multiple positions of distal-end 22a. For example, in addition to temperature sensor 64 shown in inset 60 for measuring the temperature of the aforementioned tissue, tip section 40 may comprise two additional temperature sensors 64, coupled to distal-end 22a in close proximity to electrodes 50A and 50B, so as to measure the temperature of both electrodes 50A and 50B.

In some embodiments, temperature sensor 66 may comprise a thermocouple (TC), but in other embodiments, at least one temperature sensor 66 may comprise and other suitable type of a temperature sensing device.

Reference is now made back to the general view of Fig. 1. Processor 41 is configured to receive the aforementioned electrical signal from contact-force sensor 66. Based on the electrical signal, processor 41 is configured to control IPG 45 to supply the one or more IRE pulses to electrodes 50A and 50B. In the context of the present disclosure and in the claims, processor 41
is configured to control, based on the received electrical signal, one or more parameters of the IRE pulses applied to the tissue. For example, based on the sensed contact force applied during the IRE ablation, between distal-end 22a and the tissue, processor 41 may control the energy, and/or amplitude and/or frequency of the applied IRE pulses. Moreover, processor 41 is configured to display, e.g., on display 27, a message or any other type of display, indicative of the sensed contact force, so that physician 30 may adjust the applied contact force, e.g., by slightly pushing or retracting distal-end 22a relative to the tissue at the ablation site.

In some embodiments, processor 41 is configured to hold at least a temperature threshold, and to control the supply of the one or more IRE pulses, e.g., to electrodes 50A and 50B, based on a comparison between the temperature measured by temperature sensor 66 and the temperature threshold.

This particular configuration of system 20 is shown by way of example, in order to illustrate certain problems that are addressed by embodiments of the present invention and to demonstrate the application of these embodiments in enhancing the performance of such an IRE ablation system. Embodiments of the present invention, however, are by no means limited to this specific sort of example system, and the principles described herein may similarly be applied to other sorts of ablation systems provided they fall within the scope of the appended claims.

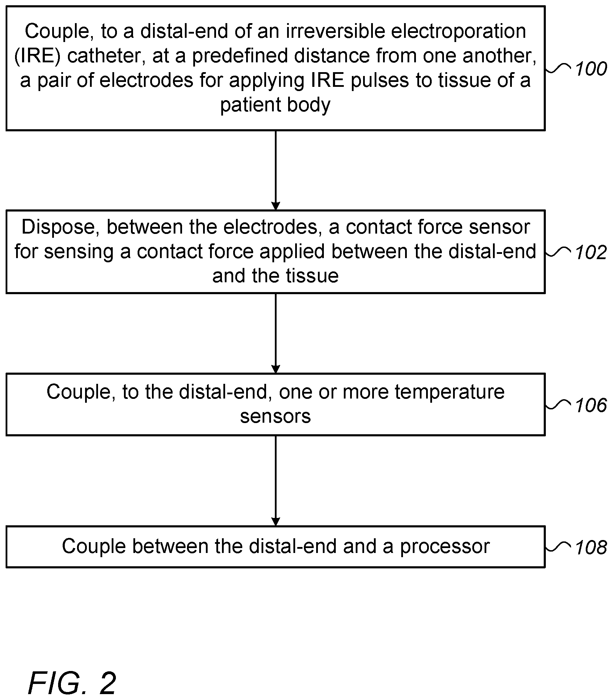

Fig. 2 is a flow chart that schematically illustrates a method for producing an IRE focal catheter having contact-force sensor 66 and temperature sensor 64, in accordance with an embodiment of the present invention. The method begins at an electrodes coupling step 100, with coupling electrodes 50A and 50B to distal-end 22a of an IRE catheter, such as catheter 21. In some embodiments, electrodes 50A and 50B are fitted at a predefined distance from one another, and are configured for applying IRE pulses to the target tissue at the ablation site of heart 26, or to tissue any other organ in the body of patient 28.

At a contact-force sensor disposing step 102, contact-force sensor 66 is disposed and fitted on tip section 40 of distal-end 22a, between electrodes 50A and 50B. As described in inset 60 of Fig. 1 above, contact-force sensor 66 is configured for sensing the contact force applied between distal-end 22a and the target tissue of heart 26, and to produce the electrical signal indicative of the sensed contact force.

At a temperature sensor coupling step 106, one or more temperature sensors 64 are coupled to tip section 40 of distal-end 22a, at predefined positions, so as to measure the temperature and to produce the temperature signal indicative of the sensed temperature, as described in Fig. 1 above.

At a processor coupling step 108 that terminates the method, distal-end 22a is coupled, via catheter 21, to processor 41 of console 24. In some embodiments, in step 108, processor 41 is electrically connected to several components of catheter 21, such as but not limited to: contact-force sensor 66, one or more temperature sensors 64, and position sensor 39.

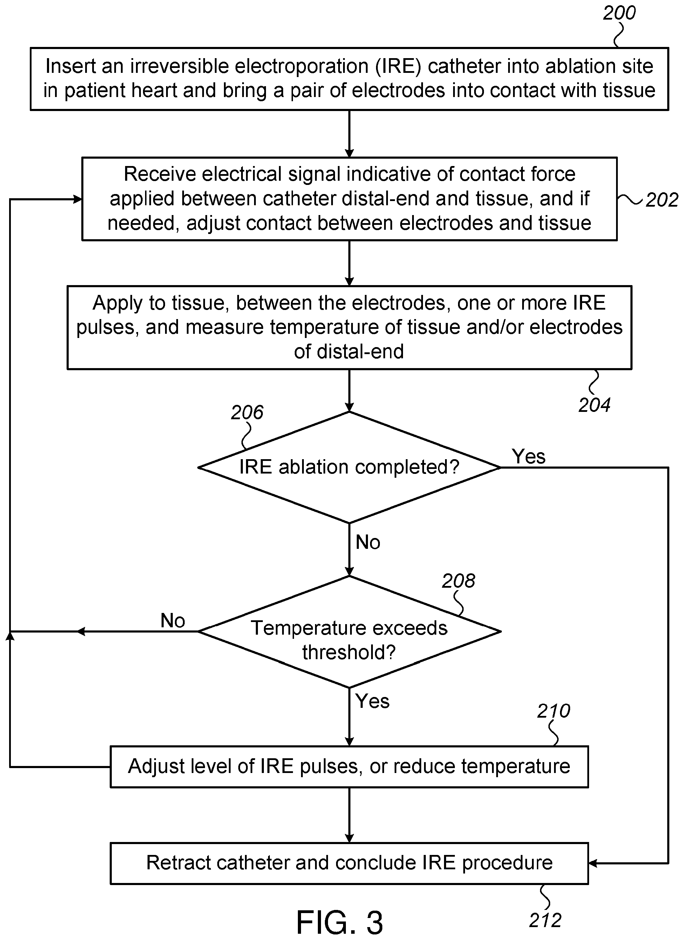

Fig. 3 is a flow chart that schematically illustrates a non-claimed method for controlling one or more IRE pulses applied to target tissue of heart 26, based on signals indicative of contact-force and temperature measured during IRE ablation procedure, using a system in accordance with an embodiment of the present invention.

The method begins at an IRE catheter insertion step 200, with inserting tip section 40, which is located at distal-end 22a of catheter 21, into the ablation site in heart 26 and bringing the pair of electrodes 50A and 50B into contact with the target tissue. As described in Fig. 1 above, tip section 40 comprises at least electrodes 50A and 50B, configured to receive IRE pulses from IPG 45 and to apply the IRE pulses to the target tissue of heart 26. In some embodiments, tip section 40 further comprises one or more contact-force sensors 66, and one or more temperature sensors 64, such as thermocouples or any other suitable type of one or more temperature sensing devices.

At a contact-force signal receiving step 202, processor 41 receives the electrical signal, which is indicative of the contact force applied between distal-end 22a and the target tissue of heart 26. In case the measured contact force is not within the specified level thereof (e.g., between about 5 gram force (grf) and 25 grf), processor 41 may display, e.g., on display 27, a message to physician to adjust the contact force applied between distal-end 22a, having electrodes 50A and 50B, and target tissue. In other embodiments, in case the measured contact force is not within the contact-force specified level, the processor may prevent the physician from applying the IRE pulses to the tissue.

At an IRE pulse applying step 204, after verifying that the applied contact force is within the specified level of the IRE procedure, processor 41 controls IPG 45 to produce one or more IRE pulses. As described in Fig. 1 above, electrodes 50A and 50B are configured to receive the one or more IRE pulses from IPG 45, and to apply, between electrodes 50A and 50B one or more bipolar IRE pulse to the target tissue. In some embodiments, processor 41 is configured to receive from one or more temperature sensors 64 one or more temperature signals indicative of the temperature measured, by temperature sensors 64, during the IRE ablation procedure. The measured temperature may comprise tissue temperature, electrodes temperature and temperature of any other component of catheter 21.

At a first decision step 206, physician 30 checks whether or not the IRE ablation has been completed. If yes, the method proceeds to a catheter retracting step 212, with physician retracting distal-end 22a of catheter 21 out of heart 26, and the IRE procedure is concluded.

If the IRE ablation has not been completed, at a second decision step 208, processor 41 compares between the temperature measured at step 204, and a temperature threshold that processor 41 holds, as described in Fig. 1 above. Based on the comparison, processor 41 checks whether or not the measured temperature exceeds the temperature threshold. If the measured temperature exceeds the temperature threshold, the method proceeds to a pulse-level adjusting step 210, in which processor 41 and/or physician 30 may adjust the number of IRE pulse applied to tissue, or reduce the number of pulses in a train, or reduce the temperature at the measured location. For example, the temperature may be reduced by waiting for a few millisecond more between trains and/or by applying irrigation (not shown) for cooling the ablated tissue and/or electrodes 50A and 50B.

Note that, based on the disclosed techniques, temperature control can be performed without modifying the IRE pulse voltage. In contrast to the IRE procedure, when applying unipolar RF power, control is typically performed by modifying the amplitude of the signal. In the present invention, however, lowering the amplitude in IRE ablation is undesirable because the voltage is key for obtaining the ablation effect.

In some embodiments, the overall energy applied to tissue may be reduced by modifying the number of pulses, and/or increasing time interval between sets of IRE pulses (also referred to herein as trains), and/or controlling the number of trains of IRE pulses applied to tissue. By using one of, or any suitable combination of these power control techniques, the electrode and/or tissue heating is prevented even without using irrigation.

In case, at step 208, the measured temperature is lower than the temperature threshold, the method loops back to step 202 for starting a new set of contact-force and temperature measurements, and for applying additional one or more IRE pulses to the target tissue until the IRE ablation is completed.

Although the embodiments described herein mainly address IRE ablation of cardiac tissue, the methods and systems described herein can also be used in other applications, such as in ablating other organs of humans or other mammals and in treatment of lung cancer and liver cancer.
It will thus be appreciated that the embodiments described above are cited by way of example, and that the present invention is not limited to what has been particularly shown and described hereinabove. Rather, the scope of the present invention is defined by the appended claims.

## Claims

1. A system comprising:
an irreversible electroporation pulse generator (IPG) (45); a focal catheter (21), comprising:
an insertion tube, which is configured to insert the catheter (21) into a patient body;
first and second electrodes (50), which are coupled to a distal-end of the catheter (21) at a predefined distance from one another, and are configured to: (i) receive, via the insertion tube, one or more irreversible electroporation (IRE) 2. pulses produced by the IPG, and (ii) apply the IRE pulses, between the first and second electrodes (50), to tissue of the patient body;
a contact-force sensor (66), which is disposed between the first and second electrodes (50) and is configured to produce an electrical signal indicative of a contact force applied between the distal-end of the catheter and the tissue; and the system further comprising
a processor (41), which is configured, based on the electrical signal received from the contact-force sensor (66), to control the IPG to supply the one or more IRE pulses to the first and second electrodes (50).

2. The system according to claim 1, wherein the catheter comprises at least a temperature sensor (64), which is coupled to the distal-end, and is configured to produce a temperature signal, indicative of a measured temperature of at least one of the distal-end and the tissue.

3. The system according to claim 2, wherein the temperature sensor (64) comprises a thermocouple.

4. The system according to claim 2, wherein the processor (41) is configured to: (i) hold a temperature threshold, and (ii) control the supply of the one or more IRE pulses to the first and second electrodes (50), based on a comparison between the measured temperature and the temperature threshold.

5. The system according to claim 1, wherein the first and second electrodes (50) are coupled along an axis of the catheter.

6. A method for producing a focal catheter, the method comprising:
coupling, to a distal-end of a catheter (21) at a predefined distance from one another, first and second electrodes (50) configured to receive one or more irreversible electroporation (IRE) pulses, and to apply the IRE pulses, between the first and second electrodes (50), to tissue of a patient body;
disposing between the first and second electrodes (50), a contact-force sensor (66) configured to produce an electrical signal indicative of a contact force applied between the distal-end of the catheter and the tissue; and
connecting to the catheter: (i) an IRE pulse generator (IPG) configured to supply the one or more IRE pulses, and (ii) a processor (41) configured to control, based on the electrical signal received from the contact-force sensor, the one or more IRE pulses supplied by the IPG to the first and second electrodes (50).

7. The method according to claim 6, and comprising coupling to the distal-end, at least a temperature sensor (64), for produce a temperature signal, indicative of a measured temperature of at least one of the distal-end and the tissue.

8. The method according to claim 6, wherein coupling the temperature sensor (64) comprises coupling a thermocouple.

9. The method according to claim 6, wherein coupling the first and second electrodes (50) comprises coupling the first and second electrodes (50) along an axis of the catheter.

## Patentansprüche

1. System, umfassend:
einen Generator (IPG) (45) von Impulsen für irreversible Elektroporation; einen fokalen Katheter (21), umfassend:
einen Einführschlauch, der konfiguriert ist, um den Katheter (21) in einen Patientenkörper einzuführen;
eine erste und eine zweite Elektrode (50), die in einem vordefinierten Abstand voneinander mit einem distalen Ende des Katheters (21) gekoppelt sind und konfiguriert sind zum: (i) Empfangen, über den Einführschlauch, eines oder mehrerer Impulse für irreversible Elektroporation (IRE), die durch den IPG erzeugt werden, und (ii) Anlegen der IRE-Impulse, zwischen der ersten und der zweiten Elektrode (50), an das Gewebe des Patientenkörpers;
einen Kontaktkraftsensor (66), der zwischen der ersten und der zweiten Elektrode (50) angeordnet ist und konfiguriert ist, um ein elektrisches Signal zu erzeugen, das eine Kontaktkraft angibt, die zwischen dem distalen Ende des Katheters und dem Gewebe angelegt wird; und das System ferner umfassend einen Prozessor (41), der konfiguriert ist, um basierend auf dem elektrischen Signal, das von dem Kontaktkraftsensor (66) empfangen wird, den IPG zu steuern, um den einen oder die mehreren IRE-Impulse der ersten und der zweite Elektrode (50) zuzuführen.

2. System nach Anspruch 1, wobei der Katheter mindestens einen Temperatursensor (64) umfasst, der mit dem distalen Ende gekoppelt ist, und konfiguriert ist, um ein Temperatursignal zu erzeugen, das eine gemessene Temperatur von mindestens einem des distalen Endes und des Gewebes angibt.

3. System nach Anspruch 2, wobei der Temperatursensor (64) ein Thermoelement umfasst.

4. System nach Anspruch 2, wobei der Prozessor (41) konfiguriert ist zum: (i) Halten eines Temperaturschwellenwerts und (ii) Steuern der Zufuhr des einen oder der mehreren IRE-Impulse zu der ersten und der zweiten Elektrode (50) basierend auf einem Vergleich zwischen der gemessenen Temperatur und dem Temperaturschwellenwert.

5. System nach Anspruch 1, wobei die erste und die zweite Elektrode (50) entlang einer Achse des Katheters gekoppelt sind.

6. Verfahren zum Erzeugen eines fokalen Katheters, das Verfahren umfassend:
Koppeln, an ein distales Ende eines Katheters (21) in einem vordefinierten Abstand voneinander, der ersten und der zweiten Elektrode (50), die konfiguriert sind, um einen oder mehrere Impulse für irreversible Elektroporation (IRE) zu empfangen und um die IRE-Impulse zwischen der ersten und der zweiten Elektrode (50) an Gewebe eines Patientenkörpers anzulegen;
Anordnen, zwischen der ersten und der zweiten Elektrode (50), eines Kontaktkraftsensors (66), der konfiguriert ist, um ein elektrisches Signal zu erzeugen, das eine Kontaktkraft angibt, die zwischen dem distalen Ende des Katheters und dem Gewebe angelegt wird; und
Anschließen an den Katheter: (i) eines IRE-Impulsgenerators (IPG), der konfiguriert ist, um den einen oder die mehreren IRE-Impulse zuzuführen, und (ii) eines Prozessors (41), der konfiguriert ist, um basierend auf dem elektrischen Signal, das von dem Kontaktkraftsensor empfangen wird, den einen oder die mehreren IRE-Impulse zu steuern, die durch den IPG der ersten und der zweiten Elektrode (50) zugeführt werden.

7. Verfahren nach Anspruch 6, und umfassend das Koppeln von mindestens einem Temperatursensor (64) mit dem distalen Ende zum Erzeugen eines Temperatursignals, das eine gemessene Temperatur von mindestens einem des distalen Endes und des Gewebes angibt.

8. Verfahren nach Anspruch 6, wobei das Koppeln des Temperatursensors (64) das Koppeln eines Thermoelements umfasst.

9. Verfahren nach Anspruch 6, wobei das Koppeln der ersten und der zweiten Elektrode (50) das Koppeln der ersten und der zweiten Elektrode (50) entlang einer Achse des Katheters umfasst.

## Revendications

1. Système comprenant :
un générateur d'impulsions d'électroporation irréversible (IPG) (45) ; un cathéter focal (21) comprenant :
un tube d'insertion, qui est conçu pour insérer le cathéter (21) dans le corps de patient ;
une première et une seconde électrodes (50), qui sont accouplées à une extrémité distale du cathéter (21) à une distance prédéfinie l'une de l'autre, et sont configurées pour : (i) recevoir, par l'intermédiaire du tube d'insertion, une ou plusieurs impulsions d'électroporation irréversible (IRE) produites par l'IPG, et (ii) appliquer les impulsions IRE, entre les première et seconde électrodes (50), au tissu du corps du patient ;
un capteur de force de contact (66), qui est disposé entre la première et la seconde électrode (50) et est configuré pour produire un signal électrique indiquant une force de contact appliquée entre l'extrémité distale du cathéter et le tissu ; et le système comprenant en outre un processeur (41), qui est configuré, sur la base du signal électrique reçu du capteur de force de contact (66), pour commander l'IPG afin de fournir la ou les impulsions IRE aux première et seconde électrodes (50).

2. Système selon la revendication 1, dans lequel le cathéter comprend au moins un capteur de température (64), qui est accouplé à l'extrémité distale, et est configuré pour produire un signal de température, indiquant une température mesurée d'au moins une des extrémités distales et du tissu.

3. Système selon la revendication 2, dans lequel le capteur de température (64) comprend un thermocouple.

4. Système selon la revendication 2, dans lequel le processeur (41) est configuré pour : (i) maintenir un seuil de température, et (ii) commander la fourniture de la ou des impulsions IRE aux première et seconde électrodes (50), sur la base d'une comparaison entre la température mesurée et le seuil de température.

5. Système selon la revendication 1, dans lequel les première et seconde électrodes (50) sont accouplées le long d'un axe du cathéter.

6. Procédé destiné à produire un cathéter focal, le procédé comprenant :
le couplage, à une extrémité distale d'un cathéter (21) à une distance prédéfinie l'une de l'autre, d'une première et seconde électrodes (50) configurées pour recevoir une ou plusieurs impulsions d'électroporation irréversible (IRE), et pour appliquer les impulsions IRE, entre les première et seconde électrodes (50), au tissu du corps de patient ;
entre la première et la seconde électrode (50), un capteur de force de contact (66) configuré pour produire un signal électrique indiquant une force de contact appliquée entre l'extrémité distale du cathéter et le tissu ; et
la connexion au cathéter : (i) un générateur d'impulsions IRE (IPG) configuré pour fournir la ou les impulsions IRE, et (ii) un processeur (41) configuré pour commander, sur la base du signal électrique reçu du capteur de force de contact, la ou les impulsions IRE fournies par l'IPG aux première et seconde électrodes (50).

7. Procédé selon la revendication 6, et comprenant l'accouplement à l'extrémité distale, d'au moins un capteur de température (64), pour produire un signal de température, indiquant une température mesurée d'au moins l'une des extrémités distales et du tissu.

8. Procédé selon la revendication 6, dans lequel l'accouplement du capteur de température (64) comprend l'accouplement d'un thermocouple.

9. Procédé selon la revendication 6, dans lequel l'accouplement des première et seconde électrodes (50) comprend l'accouplement des première et seconde électrodes (50) le long d'un axe du cathéter.
